# EUROPEAN PATENT APPLICATION

(11) **EP 1 491 205 A1**
(43) Date of publication of application: **29.12.2004**
(21) Application number: 03715663.5
(22) Date of filing: 31.03.2003
(51) Int. Cl.: A61K 38/00, A61K 45/00, A61K 47/42, A61P 21/02, A61P 25/00, A61P 25/14, A61P 27/02, A61P 43/00

(54) **REMEDY FOR HYPERMYOTONIA**

(30) Priority: 29.03.2002 JP 2002094698; 25.02.2003 JP 2003047191
(71) Applicant: Juridical Foundation, The Chemo-Sero-Therapeutic Research Institute, Kumamoto-shi, Kumamoto 860-8568 (JP)
(72) Inventor: KAJI, Ryuji, Otsu-shi, Shiga 520-0863 (JP); KOZAKI, Shunji, Tondabayashi-shi, Osaka 584-0071 (JP); OGUMA, Keiji, Okayama-shi, Okayama 701-0211 (JP)
(74) Representative: Bannerman, David Gardner
(86) International application number: PCT/JP2003/004097
(87) International publication number: WO 2003/082315

(57) **Abstract**

A remedy for muscle hyperactivity, comprising a purified botulinum neurotoxin as an active ingredient.

## Description

### TECHNICAL FIELD

The present invention relates to a remedy for muscle hyperactivity.

### BACKGROUND OF THE INVENTION

A botulinum toxin produced by *Clostridium botulinum* is absorbed in upper small intestine and then it dissociates into a non-toxic protein and a neurotoxin under alkaline conditions (in lymphatic vessels). The dissociated neurotoxin binds to a receptor at a nerve ending with its C-terminal domain and is endocytosed via the receptor. Subsequently, the neurotoxin specifically cleaves a protein of the neural presynaptic membrane with its zinc metalloendopeptidase activity to prevent calcium-dependent release of acetylcholine, thereby blocking neurotransmission at a synapse (Jankovic, J. et al., Curr. Opin. Neurol., 7:358-366, 1994).

A botulinum toxin is a toxin that causes a person to die by blocking systemic neurotransmission in botulinum poisoning. On the other hand, its activity is positively utilized. The botulinum toxin is used as a remedy for alleviating local muscle contraction by administering it directly in the muscle of a patient with a disease causing abnormal muscle hyperactivity, for example dystonia (Ryuji Kaji et al., "Dystonia and Botulinum Treatment", Shindantochiryo-sha, 1996). However, since the current remedies use the whole botulinum toxin (a botulinum toxin that is not separated into a neurotoxin and a non-toxic protein is hereinafter referred to as a progenitor toxin), some problems are pointed out such as 1) antibody formation is apt to be induced and 2) it takes a long time until the effect appears.

### DISCLOSURE OF THE INVENTION

An object of the present invention is to provide a remedy for muscle hyperactivity such as dystonia (diseases causing muscle hyperactivity) that is hard to induce antibody formation and shows its effect as soon as it is administered; the remedy of which active ingredient is stabilized; and a method for manufacturing the same.

Presently, treatment of dystonia with a botulinum toxin (a progenitor toxin) has a limitation that an antibody against the toxin is formed and detoxifies the toxin.

The inventors of the present invention have thought that separation of a progenitor toxin into a neurotoxin and a non-toxic protein and use of the neurotoxin alone for treatment might be able to delay antibody formation that leads to a limitation on treatment. Then mice were immunized with 100 units of a detoxified progenitor toxin and 100 units of a neurotoxin separately. As a result, the inventors have found that an antibody was formed in the case of the progenitor toxin and antibody formation was not observed in the case of the neurotoxin.

Furthermore, the inventors of the present invention have carried out an experiment to administer a progenitor toxin and a neurotoxin separately in a muscle of a rat and found out that a neurotoxin takes a shorter time period to block neurotransmission than a progenitor toxin. Accordingly, it became apparent that use of a neurotoxin can solve the problems of a progenitor toxin, such as 1) antibody formation is apt to be induced and 2) it takes a long time until the effect appears.

However, a non-toxic protein portion is said to be necessary to stabilize a neurotoxin and in fact, it is known that a neurotoxin separated from a non-toxic protein is unstable compared with a progenitor toxin (Sugii, S. et al., Infect. Immun., 16:910-914, 1977). In addition, experiments by the inventors of the present invention have showed that a separated neurotoxin lost its activity in such a short period of time that it could not be preserved as a product.

The inventors of the present invention have made efforts to find a condition under which a neurotoxin can be stably preserved. As a result, they have found that addition of human serum albumin makes it possible to preserve a neurotoxin because it had not lost its activity over a practically sufficient period of time, thereby leading to accomplishment of the present invention.

That is, the present invention relates to the followings.
1. A remedy for muscle hyperactivity comprising a purified botulinum neurotoxin as an active ingredient.
2. A remedy according to item 1, further comprising a botulinum neurotoxin-stabilizing substance.
3. A remedy according to item 2, wherein the botulinum neurotoxin-stabilizing substance is human serum albumin.
4. A remedy according to any one of items 1 to 3, wherein the botulinum neurotoxin is derived from botulinum toxin type A, B, C, D, E, or F.
5. A remedy according to any one of items 1 to 4, wherein the muscle hyperactivity is dystonia.
6. A remedy according to any one of items 1 to 4, wherein the muscle hyperactivity is a disease that needs treatment with a fast-acting remedy.
7. A method of producing the remedy as defined in item 2, comprising mixing a purified botulinum neurotoxin with human serum albumin.
8. A method of treating muscle hyperactivity, comprising administering a purified botulinum neurotoxin to a patient with muscle hyperactivity.
9. A method of treatment according to item 8, wherein the muscle hyperactivity is a disease that needs treatment with a fast-acting remedy.

### BRIEF DESCRIPTION OF THE DRAWINGS

Fig. 1 shows measurement results of changes in muscle action potential in posterior and anterior limbs of a rat by intramuscular administration (injection) of a progenitor toxin (BTX A) or a neurotoxin (NTX).
Fig. 2 shows measurement results of changes in compound muscle action potential in the left posterior limb of a rat by intramuscular administration of a progenitor toxin or a neurotoxin.
Fig. 3 shows measurement results of changes in compound muscle action potential in the right anterior limb of a rat by intramuscular administration of a progenitor toxin or a neurotoxin to the left posterior limb.

### BEST MODE FOR CARRYING OUT THE INVENTION

Various aspects of the present invention are explained below in detail.

A remedy for muscle hyperactivity of the present invention contains a purified botulinum neurotoxin as an active ingredient. Since a purified botulinum neurotoxin exerts its therapeutic effect as soon as it is administered, the remedy of the present invention can be used as a fast-acting remedy.

A purified botulinum neurotoxin as used herein means a neurotoxin that is separated from a non-toxic protein constituting a botulinum toxin. Such a neurotoxin can be obtained by a purifying process including (1) a step of decomposing a botulinum toxin (a progenitor toxin) into a neurotoxin and a non-toxic protein and (2) a step of separating the neurotoxin from the non-toxic protein. Hereinafter, purification of a neurotoxin is explained.

A progenitor toxin can be purified by combining appropriately ion exchange chromatography, gel filtration, affinity chromatography, hydrophobic chromatography, and the like. For example, type A can be purified by the method described in Sugii, S. et al., Infect. Immun., 12:1262, 1975; type B in Kozaki, S., et al., Infect. Immun., 10:750, 1974; type D in Miyazaki, S., et al., Infect. Immun., 17, 395, 1977; type E in Kitamura, M., et al., Biochem. Biophys. Acta 168:207, 1968; type F in Ohishi, I., et al., Appl. Microbiol. 29:444, 1975; and type G in Nukina, M., et al., Bakteriol. Mikrobiol. Hyg. A 268:220, 1988. Since a progenitor toxin is unstable at pH 7 or higher, purification is carried out under the condition of not exceeding pH 7.

Specifically, a progenitor toxin is concentrated from culture supernatant of *Clostridium botulinum* by methods such as ammonium sulfate precipitation and protamine treatment. Subsequently, the concentrated progenitor toxin is roughly purified by, for example, cation exchange chromatography, and fractions having toxin activity are collected. The fractions are further purified by gel filtration or affinity chromatography. Examples of affinity chromatography include a method involving: adsorbing a toxin to a β-lactose gel column; and eluting it with β-lactose. Toxin activity is measured by, for example, intraperitoneal injection in mice (a method involving: administering a toxin intraperitoneally into a mouse; and determining toxin activity from LD₅₀) and mouse 1 LD₅₀ is defined as 1 unit. In some cases, it is allowed to refer to a minimum lethal dose for causing a mouse to die as 1 MLD.

It is preferable that a progenitor toxin obtained be cleaved with a protease, preferably trypsin. It is preferable to cleave a progenitor toxin particularly if it is of type B. Trypsin can be removed by, for example, affinity chromatography thereafter.

Separation of a progenitor toxin into a neurotoxin and a non-toxic protein is carried out by adjusting the pH to pH 7 or higher. A neurotoxin separated can be purified by methods such as affinity chromatography, ion exchange chromatography, and gel filtration singly or in combination.

In some cases, a neurotoxin may be purified by carrying out column purification under alkaline conditions, for example at pH 8.0, from the beginning without a step of isolating a progenitor toxin.

The origin of a botulinum neurotoxin is not particularly limited. However, a toxin (a progenitor toxin) of *Clostridium botulinum* type A, B, C, D, E, or F is preferred as the origin.

The remedy of the present invention is preferably a pharmaceutical composition containing a purified botulinum neurotoxin and a botulinum neurotoxin-stabilizing substance.

The botulinum neurotoxin-stabilizing substance has only to be a substance that can stabilize a botulinum neurotoxin and does not impair the fast-acting ability of a botulinum neurotoxin in muscle hyperactivity therapy, under conditions for the preservation of the above-mentioned composition. Stabilization can be evaluated by: preserving a botulinum neurotoxin in the presence or absence of the substance; and comparing the toxin activity of the botulinum neurotoxin in the presence of the substance to that in the absence of the substance. Whether the fast-acting ability is impaired or not can be evaluated by: preserving a botulinum neurotoxin in the presence or absence of the substance; and comparing the above-mentioned therapeutic effect of the botulinum neurotoxin in the presence of the substance to that in the absence of the substance.

Examples of the botulinum neurotoxin-stabilizing substance include human serum albumin.

A preferable pharmaceutical composition in the present invention can be produced by a step of mixing a purified botulinum neurotoxin with human serum albumin. Accordingly, the present invention also provides a method of producing a pharmaceutical composition containing a botulinum neurotoxin, which method includes (1) a step of purifying a botulinum neurotoxin and (2) a step of mixing the botulinum neurotoxin with human serum albumin.

The step of purifying a botulinum neurotoxin can be carried out as described above. Furthermore, steps after the step of purification are not particularly limited as long as a step of mixing the botulinum neurotoxin with human serum albumin is included in the method. For example, a composition of the present invention can be produced by: dissolving a botulinum neurotoxin and a botulinum neurotoxin-stabilizing substance in a solvent; then carrying out sterile filtration; and filling the solution in an ampoule, a vial, or the like. The composition can be produced also by: dissolving a botulinum neurotoxin in a solvent in which a botulinum neurotoxin-stabilizing substance is previously dissolved; then conducting sterile filtration; and filling the solution in an ampoule or the like. Distilled water for injection, physiological saline, a 0.01 to 0.1-M phosphate buffer may be used as a solvent, and ethanol, glycerin, or the like may also be mixed if necessary.

Moreover, the composition of the present invention can be produced by: dissolving a botulinum neurotoxin and a botulinum neurotoxin-stabilizing substance in a solvent; then carrying out sterile filtration; filling the solution in a vial or the like; and conducting freeze-drying. Furthermore, the pharmaceutical composition of the present invention can be produced by: mixing a botulinum neurotoxin with a botulinum neurotoxin-stabilizing substance; and then filling the mixture sterilely in a vial or the like.

Specifically, a botulinum neurotoxin-stabilizing substance, preferably human serum albumin, more preferably human serum albumin available from the Japanese Red Cross Society of which safety in for human use is ensured, is added to a purified neurotoxin so that the final concentration becomes 0.1 to 5 mg/ml, preferably 0.5 to 2 mg/ml, and then cold storage, frozen storage, or freeze-drying is carried out.

To the remedy of the present invention, additives such as: saccharides such as mannitol, glucose, and lactose; sodium chloride; and sodium phosphate can be further added if necessary. The pH of the pharmaceutical composition according to the present invention in solution is normally 3 to 8, preferably 4 to 7, and more preferably 5 to 7.

In the remedy of the present invention, the content of the botulinum neurotoxin has only to be an amount effective for accomplishing the intended purpose of the present invention. In addition, if the remedy contains a botulinum neurotoxin-stabilizing substance, the content of the botulinum neurotoxin-stabilizing substance has only to be an amount sufficient to stabilize a botulinum neurotoxin.

A purified botulinum neurotoxin can be preserved stably by mixing it with a botulinum neurotoxin-stabilizing substance, for example, human serum albumin. Accordingly, the present invention also provides a method of preserving a botulinum neurotoxin stably, which method includes mixing a purified botulinum neurotoxin with human serum albumin and preserving the mixture, and a method of producing the pharmaceutical composition of the present invention, which method includes mixing a purified botulinum neurotoxin with human serum albumin.

The term "mixing" here means to produce a state in which a botulinum neurotoxin-stabilizing substance, for example, human serum albumin, continuously coexists with a botulinum neurotoxin. Usually, this state is accomplished by adding a botulinum neurotoxin-stabilizing substance to a botulinum neurotoxin that is in a solution state.

Examples of muscle hyperactivity include dystonias, other involuntary movements, abnormal muscle contractions, and others.

More specifically, examples of dystonias include: local dystonias (blepharospasm, oromandibular-facial-lingual dystonia, cervical dystonia, pharyngeal dystonia, task-specific dystonia, masseter dystonia, etc.); segmental dystonias (Meige syndrome, etc.); generalized dystonia (cerebral palsy, etc.); multifocal dystonia; and hemidystonia.

Examples of other involuntary movements include vocal tremor, head tremor, appendicular tremor, palatal tremor, hemifacial spasm, and tic.

Examples of abnormal muscle contractions include strabismus, nystagmus, myokymia, bruxism, dysphemia, painful muscle rigidity, muscle contraction headache, muscular backache, nerve root disorder with muscle cramps, spasticity, spastic bladder, achalasia, anismus, and detrusor sphincter dyssynergia.

Examples of others include kerato conjunctiva protection and facelift.

Muscle hyperactivity is preferably dystonia.

Muscle hyperactivities treated by the remedy of the present invention are preferably diseases that require quick suppression of muscle hyperactivity, that is, diseases that require treatment with a fast-acting remedy. Examples of those muscle hyperactivities include muscle hyperactivity for which a remedy is administered while the dosage is adjusted until the effective dose is determined and systemic muscle hyperactivity for which treatment is carried out by accumulating the effect of a remedy cumulatively. Examples of systemic muscle hyperactivity include generalized dystonia and systemic spasticity.

The remedy of the present invention is administered in an amount effective for treatment. When the remedy is administered to a human being, the way of administration is preferably local administration, more preferably intramuscular injection; however, administration methods for delivering the remedy to the entire body are not excluded. Moreover, the administration timing and dosage are not limited and vary depending on severity of symptoms and the like. The administration dosage varies depending on severity of symptoms, age, sex, weight, the way of administration, and the like. For instance, for an adult, 1 to 50 units, preferably 5 to 300 units are injected intramuscularly once. The term "1 unit" here means the amount of a toxin (1 LD₅₀) that causes half of the mice to die when the toxin is administered intraperitoneally to the mice.

The present invention provides a method of treating muscle hyperactivity characterized by administering a purified botulinum neurotoxin to a patient with muscle hyperactivity and use of a purified botulinum neurotoxin in the manufacture of a remedy for muscle hyperactivity. Purified botulinum toxins, muscle hyperactivity, administration methods, production methods, and the like are as explained above.

### EXAMPLES

The present invention is explained in further detail by the following examples, but the present invention is not limited thereto.

### EXAMPLE 1 Purification and study on stability of botulinum type B neurotoxin

### (1) Purification of botulinum type B neurotoxin

Culture supernatant of *Clostridium botulinum* type B (Lamanna strain) cultured by the dialysis tube culture method (Inoue, K., et al., Infect. Immun. 64:1589, 1996) was salted out with 60% saturated ammonium sulfate. A pellet thus obtained was dialyzed against a 50-mM phosphate buffer solution (pH 6.0) and was subjected to protamine treatment (Kozaki, S., et al., Infect. Immun., 10:750, 1974). Then, rough purification was carried out on the protamine-treated product on a cation exchange chromatography column equilibrated with a 50-mM sodium acetate buffer solution (pH 4.2) (Inoue, K., et al., Infect. Immun. 64:1589, 1996). Fractions having toxin activity were collected and dialyzed against a 10-mM phosphate buffer solution (pH 6.0), and then the dialyzed product was applied on a β-lactose gel (manufactured by E-Y Laboratories, Inc.) column equilibrated with the phosphate buffer solution. Botulinum L toxin that adsorbs to the column was eluted with the phosphate buffer solution containing 0.2 M lactose, so that the toxin was purified.

Botulinum L toxin thus obtained was mixed with trypsin (manufactured by Sigma Co.) in a protein ratio of toxin:enzyme = 100:1 and the mixture was allowed to react at 37°C for an hour. Then, affinity chromatography with a β-lactose gel column was carried out again under the same conditions as those described above to remove trypsin. Botulinum L toxin thus obtained was dialyzed against a 10-mM phosphate buffer solution of which pH was adjusted to 8.0, and the dialyzed product was applied on a β-lactose gel column equilibrated with the phosphate buffer solution. Fractions that were not adsorbed to the column were collected to thereby obtain a botulinum neurotoxin.

### (2) Study on stability of botulinum type B neurotoxin

Toxin activity was measured by administering a stepwise dilution series of botulinum type B neurotoxin subjected to filter sterilization with a 0.45 µm filter intraperitoneally to mice. Then, the toxin was diluted with 20-mM sodium phosphate buffer solutions with pH 6.0, 7.0, and 8.0, respectively so that the concentration of each solution became 500 MLD/0.25 ml, and then each solution was dispensed into sterile tubes. The term "1 MLD" here means a minimum administration dose that causes a ddy mouse (female, 20 g) to die when the toxin is administered intraperitoneally to the mouse.

Furthermore, human serum albumin which was diluted with each buffer solution with each pH so that the concentration became 1 mg/ml, was added to half of the dispensed tubes, and each buffer solution with each pH was added to the rest (the final concentration was 500 MLD of botulinum neurotoxin and 0.25 mg of human serum albumin/0.5 ml/tube). Each tube containing a prepared composition was subjected to cold preservation at 4°C, frozen preservation at -80°C, or preservation at 4°C after freeze-drying. Samples were picked out periodically and diluted with physiological saline, and then lethal activity for a mouse was measured.

As a result, as shown in Table 1, in the group with no addition of human serum albumin in the samples after 40 days from the beginning of the preservation, the activity had been already lost. On the other hand, in the group with addition of human serum albumin, decrease in the activity from 500 MLD was not observed under any pH conditions and any preservation conditions. Moreover, decrease in the activity was not observed in the samples after 90 days except that decrease in the activity down to 300 MLD was observed in the freeze-dried sample of pH 8.0. 4°C, -80°C, and F.D. in Table 1 refer to cold preservation at 4°C, frozen preservation at -80°C, and preservation at 4°C after freeze-drying, respectively.

**Table 1**

| After 40 days | | | | |
|---|---|---|---|---|
| | | 4°C | -80°C | F.D. |
| Albumin 250 µg | pH 6.0 | 500 MLD | 500 MLD | 500 MLD |
| | pH 7.0 | 500 MLD | 500 MLD | 500 MLD |
| | pH 8.0 | 500 MLD | 500 MLD | 500 MLD |
| No albumin added | pH 6.0 | < 1 MLD | 1 MLD | 1 MLD |
| | pH 7.0 | < 1 MLD | 1 MLD | < 1 MLD |
| | pH 8.0 | < 1 MLD | 1 MLD | < 1 MLD |

| After 90 days | | | | |
|---|---|---|---|---|
| | | 4°C | -80°C | F.D. |
| Albumin 250 µg | pH 6.0 | 500 MLD | 500 MLD | 500 MLD |
| | pH 7.0 | 500 MLD | 500 MLD | 500 MLD |
| | pH 8.0 | 500 MLD | 500 MLD | 300 MLD |

Furthermore, the longer-term stability was studied in a similar manner.

As a result, as shown in Table 2, the activity had been lost in a month from the beginning of the preservation under all the preservation conditions in the group with no addition of human serum albumin.

On the other hand, in the group with addition of human serum albumin, decrease in the activity had not been observed for 6 months in all of the samples preserved at 4°C and -80°C, and only a little decrease in the activity was observed after 12 months. In addition, in the freeze-dried samples (F.D.), a little decrease in the activity was observed after 3 months under the preservation condition at pH 8.0, and after 6 months, decrease in the activity was observed in all of the samples.

**Table 2**

| Group with addition of human serum albumin | | | | | |
|---|---|---|---|---|---|
| Preservation conditions | pH of buffer solution | Toxicity (MLD/0.5 ml) | | | |
| | | Preservation period (months) | | | |
| | | 1 | 3 | 6 | 12 |
| 4°C | 6.0 | 500 | 500 | 500 | 500 |
| | 7.0 | 500 | 500 | 500 | 500 |
| | 8.0 | 500 | 500 | 500 | 100 |
| - 80°C | 6.0 | 500 | 500 | 500 | 400 |
| | 7.0 | 500 | 500 | 500 | 500 |
| | 8.0 | 500 | 500 | 500 | 500 |
| F.D. | 6.0 | 500 | 500 | 250 | 100 |
| | 7.0 | 500 | 500 | 125 | 200 |
| | 8.0 | 500 | 500 | 250 | 200 |

| Group with no addition of human serum albumin | | |
|---|---|---|
| Preservation conditions | pH of buffer solution | Toxicity (MLD/0.5 ml) |
| | | Preservation period (months) |
| | | 1 |
| 4°C | 6.0 | < 1 |
| | 7.0 | < 1 |
| | 8.0 | < 1 |
| - 80°C | 6.0 | 1 |
| | 7.0 | 1 |
| | 8.0 | 1 |
| F.D. | 6.0 | 1 |
| | 7.0 | < 1 |
| | 8.0 | < 1 |

### EXAMPLE 2 Purification and study on stability of botulinum type A neurotoxin

### (1) Purification of botulinum type A neurotoxin

M toxin of botulinum type A was purified according to the method described in Sakaguchi, G., Ohishi, I., and Kozaki, S. 1981. BIOCHEMICAL ASPECTS OF BOTULISM: Purification and oral toxicities of Clostridium botulinum progenitor toxins. pp.21-34, Lewis, G. E.(ed.), Academic Press, New York.

After botulinum M toxin was dialyzed against a 10-mM phosphate buffer solution (pH 7.5), the toxin was adsorbed on a DEAE-Sepharose column equilibrated with the buffer solution and was eluted with a 0 to 0.3-M NaCl concentration gradient of the buffer solution, to thereby separate the toxin into a neurotoxin and a non-toxic protein. The neurotoxin thus obtained was concentrated to 1 mg/ml with a YM-10 membrane (manufactured by Amicon Co.) and was dialyzed against a 50-mM phosphate buffer solution (pH 7.5). Subsequently, the neurotoxin had been preserved at -80°C until use.

### (2) Study on stability of botulinum type A neurotoxin

The preserved botulinum type A neurotoxin was diluted with a 50-mM phosphate buffer solution (pH 7.5) containing 5 mg/ml of human serum albumin. A dilution series was prepared after filter sterilization and was administered in the tail vein of a mouse, to thereby measure the amount of toxin. The neurotoxin was further diluted with a 50-mM phosphate buffer solution (pH 7.5) containing 5 mg/ml of human serum albumin so that the toxin activity became 1000 LD₅₀/ml based on the measured toxin activity. Then, the solution was dispensed in 0.4 ml portions and was frozen quickly with liquid nitrogen and was preserved at -80°C.

In addition, in order to examine the influence of pH, the neurotoxin was diluted with buffer solutions at pH 6.0, 7.5, and 9.0, dispensed respectively, and was preserved in a frozen state in the same way as described above.

The toxin activity of botulinum neurotoxins preserved under the respective conditions was measured by the mouse intraperitoneal injection method and was shown in Tables 3 and 4 in which 1 LD₅₀ was referred to as 1 unit. By preserving botulinum neurotoxins together with human serum albumin, botulinum neurotoxins can be preserved stably for over 200 days and were stable even under any conditions at pH 6 to 9.

**Table 3**

| Preservation days | LD₅₀/ml |
|---|---|
| 0 | 1625 |
| 15 | 1290 |
| 36 | 1290 |
| 74 | 1824 |
| 112 | 1448 |
| 140 | 1468 |
| 190 | 2299 |
| 209 | 1448 |

**Table 4**

| Preservation days | LD₅₀/ml | | |
|---|---|---|---|
| | pH 6.0 | pH 7.5 | pH 9.0 |
| 3 | 1328 | 1290 | 1448 |
| 10 | 1290 | 1290 | 1378 |
| 24 | 1448 | 1287 | 1152 |
| 38 | 1626 | 1290 | 1448 |

### EXAMPLE 3 Inhibitory effect on neurotransmission by botulinum type A neurotoxin in a rat

Change in muscle action potentials in the posterior limbs and anterior limbs of a rat (Wister rat) by administering 2 units of progenitor toxin or neurotoxin intramuscularly (injection), was measured. Botox manufactured by Allergan, Inc. was used as progenitor toxin, and botulinum neurotoxin type A prepared in Example 2 (the composition prepared for the stability test) was used as neurotoxin. As the unit number of the progenitor toxin, the unit number indicated in the product was adopted. As for neurotoxin, 1 LD₅₀ when the neurotoxin was administered intraperitoneally in a mouse, was defined as 1 unit.

As to muscle action potential in the posterior limbs, electrical stimulation was carried out by inserting electrodes so that lumbar vertebrae of a rat were sandwiched between the electrodes, and compound muscle action potential (CMAP) was recorded from bilateral posterior limb muscles using a Neuropack 8 (manufactured by Nihon Kohden Co.). As to muscle action potential in the anterior limbs, electrical stimulation was carried out by inserting electrodes so that cervical vertebrae of a rat were sandwiched between the electrodes, and compound muscle action potential (CMAP) was recorded from bilateral anterior limb muscles in a similar manner.

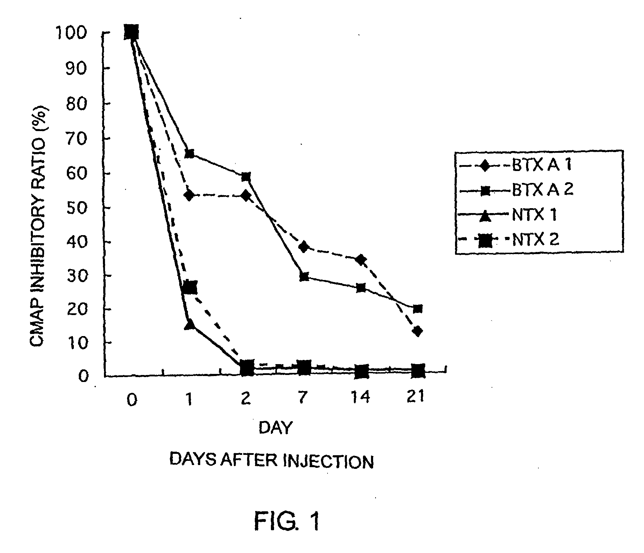

The results are shown in Fig. 1. A CMAP inhibitory ratio is an amplitude ratio when the amplitude before injection is defined as 100%. As apparent from Fig. 1, it was shown that although equal 2 units of progenitor toxin (BTX A) or neurotoxin (NTX) were administered intramuscularly, the progenitor toxin needed a few days until its effect appeared after administration, whereas the neurotoxin inhibited neurotransmission from the next day and showed its effect quickly after administration. The experiments were carried out using two rats for each group. The suffixes 1 and 2 of BTX A and NTX in the explanatory note of Fig. 1 refer to the numbers of the rats.

Furthermore, in order to confirm that neurotoxin shows its effect quickly in comparison with progenitor toxin, the effect appearance speed was compared by administering intramuscularly in the left posterior limb a unit of neurotoxin and two units of progenitor toxin separately, both of which had the equivalent potencies in terms of the lethal dose for a mouse.

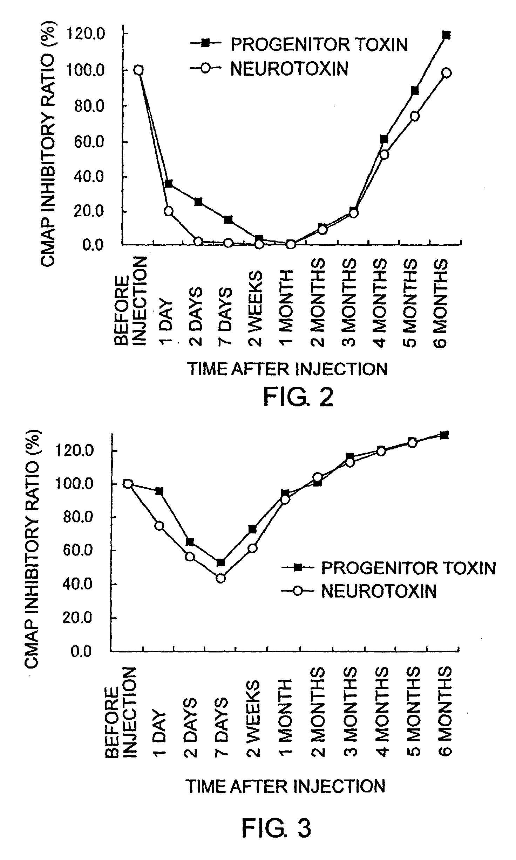

The results are shown in Figs. 2 and 3. As apparent from Fig. 2 (the data on the left posterior limb), neurotoxin inhibited neurotransmission completely from the second day since administration although the administration dose was reduced to half. It was shown that neurotoxin showed its effect quickly in comparison with progenitor toxin that required two weeks until neurotransmission was completely inhibited. Moreover, as apparent from Fig. 3 (the data on the right anterior limb), both neurotoxin and progenitor toxin inhibited neurotransmission besides that of the injected muscle, but they had no influence on the muscles except for the injected muscle in view of observation of behavior of the rats.

### EXAMPLE 4 Comparison of antigenicities of botulinum type A neurotoxin and progenitor toxin

The antigenicities of botulinum type A neurotoxin and progenitor toxin were compared. Each toxin that was diluted to 300 µg/ml had been dialyzed against 0.2% formalin and 0.1 M phosphate buffer (pH 7.0) for 301 weeks to be detoxified. Each toxin equivalent to 100 units was subcutaneously injected five times every two weeks to immunize a mouse. A blood sample was taken a month after the last immunization and the antibody titer was measured by the ELISA method using neurotoxin as the antigen.

As shown in Table 5, the antibody against toxin was formed in a mouse immunized against progenitor toxin, but antibody formation was not observed in a mouse immunized against neurotoxin. Neurotoxin was considered to have a low antigenicity compared with progenitor toxin.

**Table 5**

| Mouse No. | 100-unit progenitor toxin | 100-unit neurotoxin |
|---|---|---|
| 1. | > 10200 | < 400 |
| 2. | x 3200 | < 400 |
| 3. | x 3200 | < 400 |

### EXAMPLE 5 Purification and study on stability of botulinum type D neurotoxin

### (1) Purification of botulinum type D neurotoxin

Culture supernatant of *Clostridium botulinum* type D (D-CB16 strain) cultured by the dialysis tube culture method was salted out with 60% saturated ammonium sulfate. A pellet thus obtained was dialyzed against a 50-mM phosphate buffer solution (pH 4.0) containing 0.2 M NaCl. Then, rough purification of botulinum L toxin was carried out with an SP-Toyopearl 650S. This fraction was subjected to gel filtration on a Hiload superdex 200pg equilibrated with a 50-mM sodium phosphate buffer solution (pH 6.0) containing 0.15 M NaCl and then was purified on a Mono S column.

Botulinum L toxin thus purified was dialyzed overnight against a 20-mM Tris-HCl buffer solution (pH 8.8) containing 0.4 M NaCl and neurotoxin was purified on a superdex 200pg gel filtration column equilibrated with the buffer solution.

### (2) Study on stability of botulinum type D neurotoxin

Toxin activity was measured by administering a stepwise dilution series of botulinum type D neurotoxin subjected to filter sterilization with a 0.45 µm filter intraperitoneally to mice. Then, the toxin was diluted with 20-mM sodium phosphate buffer solutions with pH 6.0, 7.0, and 8.0, respectively so that the concentration of each solution became 500 MLD/0.25 ml, and then each solution was dispensed into sterile tubes.

Furthermore, human serum albumin which was diluted with each buffer solution with each pH so that the concentration became 1 mg/ml, was added to half of the dispensed tubes, and each buffer solution with each pH was added to the rest (the final concentration was 500 MLD of botulinum neurotoxin and 0.25 mg of human serum albumin/0.5 ml/tube). Each tube containing a prepared composition was subjected to cold preservation at 4°C, or frozen preservation at -80°C. Samples were picked out periodically and diluted with physiological saline, and then lethal activity for a mouse was measured.

As a result, as shown in Table 6, in the group with no addition of human serum albumin in the samples after a month from the beginning of the preservation, the activity had been already lost. On the other hand, in the group with addition of human serum albumin, decrease in the activity from 500 MLD was not observed under any pH conditions and any preservation conditions for 6 months.

**Table 6**

| Group with addition of human serum albumin | | | | |
|---|---|---|---|---|
| Preservation conditions | pH of buffer solution | Toxicity (MLD/0.5 ml) | | |
| | | Preservation period (months) | | |
| | | 1 | 3 | 6 |
| 4°C | 6.0 | 500 | 500 | 500 |
| | 7.0 | 500 | 500 | 500 |
| | 8.0 | 500 | 500 | 500 |
| - 80°C | 6.0 | 500 | 500 | 500 |
| | 7.0 | 500 | 500 | 500 |
| | 8.0 | 500 | 500 | 500 |

| Group with no addition of human serum albumin | | |
|---|---|---|
| Preservation conditions | pH of buffer solution | Toxicity (MLD/0.5 ml) |
| | | Preservation period (months) |
| | | 1 |
| 4°C | 6.0 | < 1 |
| | 7.0 | < 1 |
| | 8.0 | < 1 |
| - 80°C | 6.0 | 1 |
| | 7.0 | 1 |
| | 8.0 | 1 |

### EXAMPLE 6 Comparison between safety of neurotoxin and that of progenitor toxin

Neurotoxin and progenitor toxin were administered intramuscularly respectively to compare an effective dose and a lethal dose, so that safety of neurotoxin and that of progenitor toxin were compared.

Botulinum type A neurotoxin prepared in Example 2 (the composition prepared for the stability test) was used as neurotoxin and Botox manufactured by Allergan, Inc. was used as progenitor toxin. As for neurotoxin, 1 LD₅₀ when the neurotoxin was administered intraperitoneally in a mouse, was defined as 1 unit. As the unit number of the progenitor toxin, the unit number indicated in the product was adopted.

Concerning the effective dose, neurotoxin or progenitor toxin was administered intramuscularly in the posterior limbs of a Wister rat and CMAP of the injected muscles after two days was measured in the same manner as in Example 3 and an administration dose that inhibited CMAP by 50% was defined as the effective dose. In addition, concerning the lethal dose, neurotoxin or progenitor toxin was administered intramuscularly in four Wister rats to define an administration dose that caused even one rat to die after five days as the lethal dose.

As a result, the effective dose and lethal dose of neurotoxin were 0.01 unit and 20 units, respectively. The safety margin was 200-fold. On the other hand, the effective dose and lethal dose of progenitor toxin were 0.05 unit and 50 units, respectively. The safety margin was 100-fold. This result revealed that neurotoxin had a wide safety margin compared with progenitor toxin.

### INDUSTRIAL APPLICABILITY

The present invention provides a remedy for muscle hyperactivity that is hard to induce antibody formation and shows its effect soon after administration. In addition, according to a preferable embodiment of the present invention, a pharmaceutical preparation that can be stably preserved can be provided.

## Claims

1. A remedy for muscle hyperactivity, comprising a purified botulinum neurotoxin as an active ingredient.

2. A remedy according to claim 1, further comprising a botulinum neurotoxin-stabilizing substance.

3. A remedy according to claim 2, wherein the botulinum neurotoxin-stabilizing substance is human serum albumin.

4. A remedy according to any one of claims 1 to 3, wherein the botulinum neurotoxin is derived from botulinum toxin type A, B, C, D, E, or F.

5. A remedy according to any one of claims 1 to 4, wherein the muscle hyperactivity is dystonia.

6. A remedy according to any one of claims 1 to 4, wherein the muscle hyperactivity is a disease that needs treatment with a fast-acting remedy.

7. A method of producing the remedy as defined in claim 2, comprising mixing a purified botulinum neurotoxin with human serum albumin.

8. A method of treating muscle hyperactivity, comprising administering a purified botulinum neurotoxin to a patient with muscle hyperactivity.

9. A method of treatment according to claim 8, wherein the muscle hyperactivity is a disease that needs treatment with a fast-acting remedy.
